# FASCICULE DE BREVET EUROPEEN

(11) **EP 2 431 359 B1**
(45) Date de publication et mention de la délivrance du brevet: **03.06.2020**
(21) Numéro de dépôt: 11191740.7
(22) Date de dépôt: 12.07.2007
(51) Int. Cl.: C07D 279/20, C07D 279/22, A61K 31/5415, A61P 31/00, A61P 25/00, A61P 7/00

(54) **Procédé de préparation de composés diaminophenothiazinium**
Verfahren zur Herstellung von Diaminophenothiaziniumverbindungen
Process for preparing diaminophenothiazinium compounds

(30) Priorité: 12.07.2006 FR 0606330
(43) Date de publication de la demande: 21.03.2012
(62) Demande divisionnaire de: 07803863.5
(73) Titulaire: Provepharm Life Solutions, 13013 Marseille (FR)
(72) Inventeur: Feraud, Michel, 13013 Marseille (FR); Sayah, Babak, 13013 Marseille (FR)
(74) Mandataire: Corizzi, Valérie

(56) Documents cités:
- EP-A2- 2 057 136
- WO-A-2005/054217
- WO-A2-2006/032879
- FR-A1- 2 810 318
- US-A- 4 003 892
- DATABASE BEILSTEIN CROSSFIRE Beilstein Institute of Organic Chemistry; Reaction ID: 5575384 1899, XP002461752, & COHN, G.: ARCH.PHARM., vol. 237, 1899, page 387,
- DATABASE BEILSTEIN CROSSFIRE Beilstein Institute of Organic Chemistry; Reaction ID: 5575388 XP002461753, & COHN, G.: ARCH.PHARM., vol. 237, 1899, page 387,
- GENSLER, WALTER J. ET AL: "Hydrolysis and autoxidation of N-benzoylleucomethylene blue", JOURNAL OF ORGANIC CHEMISTRY , 31(7), 2324-30 CODEN: JOCEAH; ISSN: 0022-3263, 1966, XP002461751,
- NERENBERG C ET AL: "Purification of Thionin, Azure A, Azure B and Methylene Blue", STAIN TECHNOLOGY, WILLIAMS AND WILKINS, BALTIMORE, US, vol. 38, no. 2, 1 March 1963 (1963-03-01), pages 75-84, XP009176224, ISSN: 0038-9153
- MARSHALL P N ET AL: "The Purification of Methylene Blue and Azure B by Solvent Extraction and Crystallization", STAIN TECHNOLOGY, WILLIAMS AND WILKINS, BALTIMORE, US, vol. 50, no. 6, 1 January 1976 (1976-01-01), pages 375-381, XP009176225, ISSN: 0038-9153

## Description

La présente invention a pour objet un nouveau procédé de préparation de composés du type diaminophénothiazinium, en particulier un procédé de purification de ces composés. Elle concerne en particulier le bleu de méthylène, et la demande décrit les produits résultant de ce procédé dont le degré de pureté est plus élevé que ceux connus de l'art antérieur. La demande décrit encore l'utilisation de ces composés pour la préparation de médicaments.

Le chlorure de méthylthioninium, également connu sous les noms de bleu de méthylène ou chlorure de 3,7-bis(diméthylamino)phénothiazine-5-ylium, est un composé organique répondant à la formule ci-dessous :

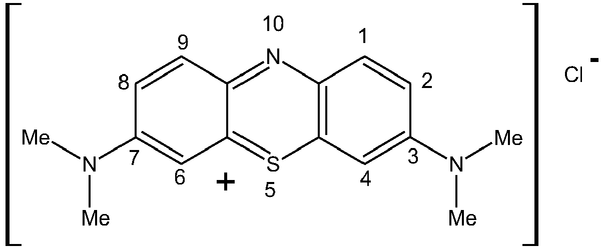

Ce composé a été utilisé de longue date comme colorant et indicateur redox, comme révélateur optique dans des systèmes biophysiques, dans des matériaux nanoporeux comme matériau séparateur, et en imagerie photoélectrochromique. Il est également connu pour ses applications comme agent antiseptique, antiinfectieux, comme antidote et comme agent de diagnostique. Il trouve des utilisations notamment en gynécologie, néonatalogie, cancérologie, oncologie, urologie, ophtalmologie et gastro-entérologie. De nouvelles utilisations dans le domaine thérapeutique sont en cours de développement, telle que la réduction des contaminants pathogéniques dans le sang (GB2373787), la prévention ou l'inhibition d'une réaction hémodynamique excessive (WO03/082296).

De nombreuses méthodes de synthèse ont été décrites pour ce composé, depuis la plus ancienne en 1877 (Brevet allemand n° 1886). Toutes ces méthodes ont en commun d'utiliser des composés métalliques dans au moins une étape de synthèse :
Le Brevet DE-1886 décrit un procédé dans lequel on fait un couplage oxydant du N,N-diméthyl-1,4-diaminobenzène avec H₂S et FeCl₃.

Fiez David et al., « Fundamental Processes of Dye Chemistry », 1949, Interscience, 308-314, décrit un procédé dans lequel le cycle thiazine est formé par traitement au dioxyde de manganèse ou au sulfate de cuivre. Ce procédé comporte encore un traitement au chlorure de zinc et au dichromate de sodium et au thiosulfate d'aluminium.

Le document WO 2005/054217 décrit des dérivés du bleu de méthylène et un procédé pour leur préparation. La méthode de préparation de ces composés utilise la phénotiazine comme produit de départ. Or toutes les méthodes de préparation connues de la phénotiazine font appel à des réactifs métalliques dont les atomes de métaux viennent chélater la phénotiazine à la fin de la synthèse. Les produits obtenus par ce procédé sont donc naturellement contaminés par des résidus métalliques, outre les contaminants organiques habituels comme l'azur B.

Le document WO 2006/032879 décrit un procédé de préparation du bleu de méthylène qui comporte une étape de réduction par du fer, une étape d'oxydation par du dichromate de sodium, une étape d'oxydation par du sulfate de cuivre.

Ces procédés requièrent d'effectuer des purifications fastidieuses et coûteuses afin de réduire les impuretés, notamment les impuretés métalliques du bleu de méthylène. Malgré les étapes de purifications ultérieures, ces différents procédés conduisent inévitablement à un bleu de méthylène comportant de nombreuses impuretés métalliques et également des impuretés organiques, notamment de l'azur B, de l'azur C et de l'azur A.

Le document WO 2006/032879 affirme pouvoir atteindre un taux d'impuretés métalliques représentant 10% du seuil maximal fixé par la pharmacopée européenne, mais d'après les exemples on constate que ce taux n'est pas obtenu simultanément pour tous les métaux et que les résultats des étapes de purification ne sont pas toujours reproductibles. Une analyse détaillée des contenus en métaux de différents bleus de méthylène disponibles commercialement est illustrée dans ce document.

La pharmacopée européenne a été modifiée récemment (avril 2006) dans le sens d'une augmentation des seuils de tolérance des impuretés métalliques car aucun producteur de bleu de méthylène n'était en mesure de produire, et encore moins de produire en quantité industrielle, un bleu de méthylène d'une qualité répondant à ses précédentes exigences.

Un premier objet de l'invention a donc été la mise au point d'un procédé de préparation du bleu de méthylène qui donne accès à un bleu de méthylène d'une grande pureté, notamment qui comporte un très faible taux d'impuretés métalliques et organiques, qui soit extrapolable à l'échelle industrielle dans des conditions économiques satisfaisantes, et qui ne soit pas sujet à des variations de qualité. Selon une variante, le procédé de l'invention est un procédé de purification du bleu de méthylène.

Le procédé qui a été mis au point s'applique non seulement au bleu de méthylène, mais également à d'autres dérivés de type diaminophénothiazinium.

Le procédé de l'invention est un procédé de préparation de composés répondant à formule (I) ci-dessous : 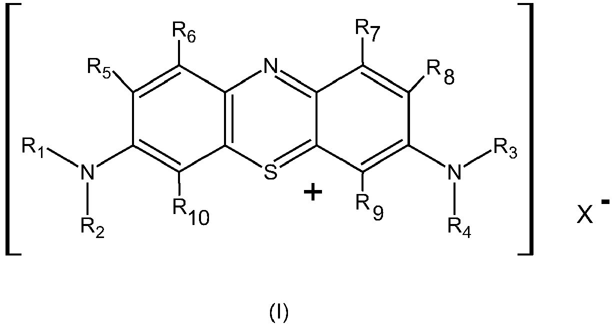 dans laquelle chacun de R₁, R₂, R₃, R₄, R₅, R₆, R₇, R₈, R₉, R₁₀ peut être choisi, indépendamment des autres, parmi le groupe constitué de :
- l'atome d'hydrogène,
- les groupements alkyle en C₁-C₆, linéaires ramifiés ou cycliques, saturés ou insaturés, éventuellement substitués par une ou plusieurs fonctions choisies parmi un atome d'halogène, une fonction alcoxy en C₁-C₆, alkyloxycarbonyle en C₁-C₆, - CONH₂,
- les groupements aryle éventuellement substitués par une ou plusieurs fonctions choisies parmi : un alkyle en C₁-C₄, un atome d'halogène, une fonction alcoxy en C₁-C₆, alkyloxycarbonyle en C₁-C₆, -CONH₂,
étant entendu que deux groupements Ri (i=1,2,...10) placés successivement sur la figure (I) peuvent être joints pour former un cycle. Par exemple R₁ avec R₅, ou R₅ avec R₆, R₇ avec R₈, R₈ avec R₃, R₃ avec R₄, R₄ avec R₉, R₁₀ avec R₂ ou R₂ avec R₁ peuvent consister en une seule chaîne alkyle éventuellement substituée de façon à former un quatrième cycle,
en outre, chacun de R₅, R₆, R₇, R₈, R₉, R₁₀ peut être choisi, indépendamment des autres, parmi les atomes d'halogène : F, Cl, Br, I,
X⁻ représente un anion organique ou inorganique.

Les anions utilisables incluent, par exemple, les anions des acides minéraux tels que par exemple l'acide chlorhydrique, l'acide bromhydrique, l'acide sulfurique, l'acide phosphorique, l'acide nitrique ; les anions des acides organiques tels que par exemple, l'acide acétique, l'acide trifluoroacétique, l'acide oxalique, l'acide tartrique, l'acide succinique, l'acide maléique, l'acide fumarique, l'acide gluconique, l'acide citrique, l'acide malique, l'acide ascorbique, l'acide benzoïque, ils incluent également OH⁻.

Ce procédé est caractérisé en ce qu'il comporte préférentiellement au moins une étape au cours de laquelle un composé de formule (II) : 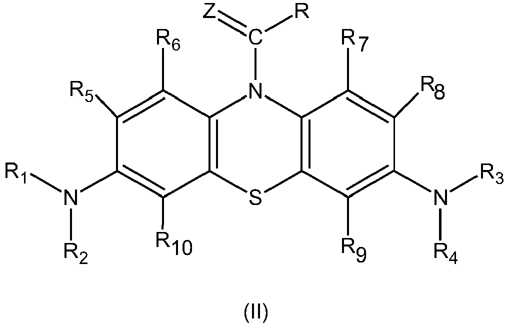 est soumis à une étape de purification dans des conditions permettant de séparer des composés métalliques des composés de formule (II), les groupements R₁, R₂, R₃, R₄, R₅, R₆, R₇, R₈, R₉, R₁₀ ayant la même définition que dans la formule (I) et R représente un groupement choisi parmi :
- un groupement phényle ou benzyle, éventuellement substitués par une ou plusieurs fonctions choisies parmi : un alkyle en C₁-C₄, un atome d'halogène, un halogénoalkyle en C₁-C₄, un groupement nitro,
- un groupement alkyle en C₁-C₈, linéaire, ramifié ou cyclique,
- un groupement alkyl amino en C₁-C₈,
- un groupement alcoxy en C₁-C₈,
- un groupement phényloxy ou benzyloxy éventuellement substitués sur le noyau aromatique par une ou plusieurs fonctions choisies parmi : un alkyle en C₁-C₄, un atome d'halogène, un halogénoalkyle en C₁-C₄, un groupement nitro,

Z représente un atome choisi parmi O et S.

La purification des composés de formule (II) est faite dans des conditions permettant de séparer les composés métalliques des composés de formule (II) : filtration sur un support susceptible de retenir les composés métalliques, cristallisation dans un solvant approprié ou toute autre méthode connue de l'homme du métier.

Lorsque la purification est faite par filtration sur un support susceptible de retenir les composés métalliques, un tel support peut être choisi parmi : un gel de silice, un gel d'alumine (neutre, basique ou acide), une diatomite éventuellement modifiée, de la célite, une membrane microporeuse, les résines greffées par des groupements capteurs de métaux et les fibres greffées par des groupements capteurs de métaux, tels que des fonctions thiol, acide carboxylique, amine tertiaire, ou tout autre support ayant la propriété de retenir les métaux. Parmi les fibres greffées, on peut citer notamment les produits commercialisés par la société Johnson Matthey sous la marque Smopex ®. Parmi les diatomites, on peut citer les produits commercialisés par la société CECA sous la marque Clarcel ®.

Le composé de formule (II) peut être obtenu à partir du composé de formule (I), par réduction du composé de formule (I) puis par réaction de la fonction amine du cycle phénothiazinium avec un groupement protecteur approprié R-CZ-Y dans lequel R et Z ont la même définition que ci-dessus et Y représente un groupe partant choisi parmi : un atome d'halogène tel que F, Cl, I, Br, un groupement alcoxy en C₁-C₆, un groupement - OCOR (anhydride), un groupement hydroxyle, éventuellement en présence d'un activateur du type dicyclohexylcarbodiimide (DCC). Avantageusement R est choisi parmi un groupement phényle, un groupement toluyl.

Lorsque le composé de formule (II) est obtenu à partir du composé de formule (I), le procédé global est une purification du composé de formule (I). Toutefois, le composé de formule (II) peut être obtenu par d'autres procédés qui n'utilisent pas le produit (I) comme produit de départ.

Certains composés de formule (II) tels que le benzoyl leuco bleu de méthylène sont disponibles commercialement.

Le composé représenté par la formule (I), peut être représenté par plusieurs structures résonnantes équivalentes. A titre d'illustration et de façon non limitative, on fait figurer ci-dessous d'autres structures qui sont équivalentes à celle de la formule (I) :

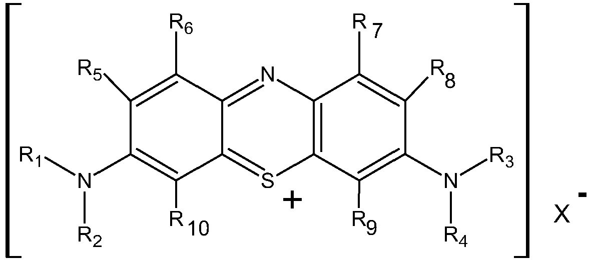

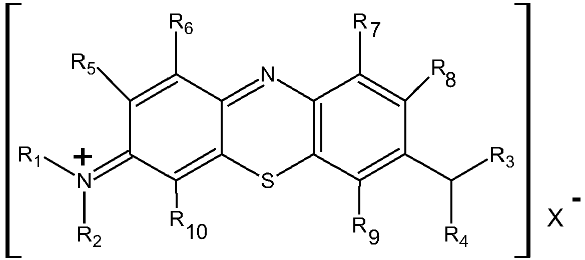

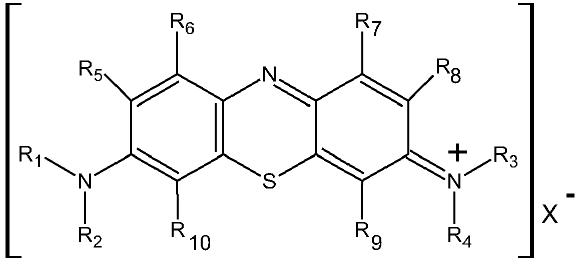

Dans la formule (I) et dans la formule (II), de préférence R₁, R₂, R₃, R₄, R₅ et R₆, R₇, R₈, R₉, R₁₀, identiques ou différents sont choisis parmi l'atome d'hydrogène et un alkyle en C₁-C₄. Avantageusement R₅, R₈, R₉ et R₁₀ représentent H.

Avantageusement encore, l'une ou plusieurs des conditions suivantes sont vérifiées :
- X représente Cl ou OH,
- R₁, R₂, R₃, R₄, identiques ou différents sont choisis parmi un atome d'hydrogène et le méthyle,
- R₆ représente un atome d'hydrogène,
- R₇ représente un atome d'hydrogène,
- Z représente O.

De façon avantageuse le composé de formule (I) est le chlorure de tétraméthylthionine ou bleu de méthylène.

Selon une autre variante le composé de formule (I) est le chlorure de diméthylthionine ou Azur A, ou le chlorure de triméthylthionine ou Azur B, ou le chlorure de monométhylthionine ou Azur C.

Selon l'invention, avantageusement, le procédé de préparation du composé de formule (I), comporte au moins une étape de purification d'un composé de formule (II), en particulier cette purification comporte au moins une étape de filtration d'un composé de formule (II) sur un support susceptible de retenir les composés métalliques, tel qu'un gel de silice, d'alumine (neutre, basique ou acide), une diatomite éventuellement modifiée, une résine fonctionnalisée par des capteurs de métaux, des fibres fonctionnalisées par des capteurs de métaux, de la célite, une membrane microporeuse ou tout autre support capable de retenir les composés métalliques.

De façon plus détaillée, selon cette variante, le composé de formule (II) est mis en solution dans un solvant approprié, on prépare un filtre avec le support de filtration que l'on introduit dans un récipient approprié, tel qu'une colonne de verre, un filtre en verre fritté ou une essoreuse industrielle. Le récipient rempli du support de filtration choisi est humidifié, préférentiellement par le même solvant que celui dans lequel est dissout le composé de formule (II).

La solution contenant le composé de formule (II) est déposée sur le filtre, la solution qui traverse le filtre est récupérée, le filtre est rincé plusieurs fois par un solvant identique ou différent de celui ayant servi à solubiliser le composé de formule (II). Les fractions éluées sont récupérées et éventuellement concentrées.

Parmi les solvants utilisables pour solubiliser les composés de formule (II) on peut citer préférentiellement : les solvants chlorés, comme par exemple le dichlorométhane ou le chloroforme, les alcools tels que isopropanol, éthanol, méthanol ou acétonitrile, acétate d'éthyle, tétrahydrofurane, ou un mélange de ces solvants.

La solution du composé de formule (II) est avantageusement d'une concentration allant de 1 g/l à 10³ g/l. Des concentrations plus faibles conduisent à utiliser des volumes de solvant trop importants avec des conséquences sur la sécurité et la taille du matériel. Des concentrations plus importantes sont difficilement envisageables en raison de la solubilité des produits.

On prévoit d'utiliser environ 0,1 à 10 kg de support de filtration par kg de produit à filtrer. On prévoit avantageusement de rincer le filtre avec 0,1 à 50 1 de solvant par kg de produit de formule (II) jusqu'à élution totale du produit de formule (II). Le procédé de l'invention présente l'avantage de débarrasser le produit de formule (II) de ses impuretés métalliques.

Lorsque l'on choisit de purifier le composé de formule (II) par cristallisation, on choisit avantageusement un solvant parmi : un alcool comme l'éthanol, un solvant chloré comme le chlorure de méthylène.

De façon avantageuse, la fabrication du composé de formule (II) se fait à partir du composé de formule (I) que l'on fait réagir avec un groupement de protection R-CZ-Y dans lequel Y est avantageusement choisi parmi : F, Cl, Br, I, un groupement alcoxy en C₁-C₆, un groupement -OCOR (anhydride), un groupement hydroxyle, éventuellement en présence d'un activateur du type dicyclohexylcarbodiimide (DCC).

La réaction se fait de façon classique en milieu basique ou neutre dans de l'eau ou dans un mélange d'eau et d'un autre solvant tel que par exemple acétonitrile, tétrahydrofurane, dichlorométhane ou tout autre solvant organique approprié.

La réaction est exothermique, et on utilise de préférence des moyens de refroidissement qui permettent de maintenir la température du mélange aux environs de la température ambiante.

Le produit de départ (I) est soit commercial, soit préparé par des méthodes connues, telles que celles décrites dans WO 2006/032879.

D'une façon générale les produits de formule (I) sont préparés par des procédés de synthèse qui font appel à l'utilisation de dérivés métalliques que l'on retrouve comme impuretés dans les produits (I). C'est le cas du bleu de méthylène, mais aussi de l'azur A, de l'azur B et de l'azur C.

Les composés de formule (I) ne peuvent pas être débarrassés de leurs impuretés métalliques et organiques de façon directe, simple et efficace. Les méthodes de l'art antérieur font appel à des recristallisations successives qui n'ont pas des rendements satisfaisants et qui conduisent à des produits dont le taux d'impuretés résiduelles est difficile à contrôler.

En outre les produits de formule (I) ont la propriété de chélater les métaux alors que les produits (II) sont non chélatants. Leur purification est donc beaucoup plus efficace que la purification directe des composés de formule (I).

Dans les différentes étapes du procédé de l'invention, on veille à employer des matériaux non métalliques, des réactifs et des solvants dépourvus de résidus métalliques de façon à ne pas introduire de contamination externe.

Après que le produit de formule (II) ait été purifié, en particulier soumis à une filtration, suivant le procédé de l'invention, on procède à une étape de déprotection de l'amine du cycle phénothiazine du composé de formule (II). Cette déprotection est faite par tout moyen connu de l'homme du métier, en évitant l'introduction de contaminants métalliques et dans des conditions évitant la dégradation du composé de formule (I). La demande décrit des moyens utilisables pour la déprotection du groupe R-CZ- : les quinones, comme par exemple la 2,3-dichloro-5,6-dicyano-1,4-benzoquinone (DDQ), HNO₃, HClO₄, I₂, HCl, H₂SO₄, H₂O₂, un traitement par des rayonnements ultraviolets. Selon l'invention, on utilise une quinone pour cette étape, et de façon très préférentielle la 2,3-dichloro-5,6-dicyano-1,4-benzoquinone. De façon avantageuse, cette réaction de déprotection est faite dans un solvant choisi parmi : l'acétate d'éthyle, l'acétonitrile, le tétrahydrofurane, l'acétone. Le solvant préféré pour cette étape est l'acétonitrile.

Des conditions de déprotection avantageuses prévoient l'emploi de 0,80 à 1,1 équivalents molaires de DDQ par rapport au composé (II), encore plus avantageusement de 0,85 à 1,05 équivalents molaires de DDQ par rapport au composé (II), avantageusement de 0,90 à 1 équivalent molaire. Préférentiellement, cette déprotection se fait à une température comprise entre -40°C et -5°C. Bien que non totalement exclues, une température plus basse aurait l'inconvénient d'allonger les durées de réaction, une température plus élevée pourrait conduire à la formation de produits secondaires.

En fonction du moyen de déprotection employé on peut être amené à faire un échange d'ions pour aboutir au composé de formule (I) comportant l'anion X⁻ souhaité. De préférence, cet échange d'ion est fait par traitement avec HCl, avantageusement dans l'acétate d'éthyle. D'autres solvants pourraient être utilisés, mais certains sont susceptibles de conduire à la formation de produits secondaires.

Les conditions de déprotection des composés de formule (II) exposées ci-dessus sont particulièrement avantageuses en ce qu'elles permettent d'aboutir à un composé de formule (I) sans introduire au cours de cette étape d'impuretés métalliques ou former d'impuretés organiques. Selon une variante de l'invention, on peut prévoir de purifier le composé de formule (II) par d'autres moyens que la filtration sur un support susceptible de retenir les métaux, comme par exemple par cristallisation dans un solvant approprié. Selon cette variante, on déprotège ensuite le composé de formule (II) à l'aide de tout moyen de déprotection n'impliquant pas l'utilisation de composés métalliques, en particulier à l'aide d'une quinone, en particulier le DDQ, de préférence dans les conditions exposées ci-dessus.

Un objet de l'invention est donc un procédé de préparation de composés répondant à formule (I) décrite ci-dessus, caractérisé en ce qu'il comporte au moins une étape de déprotection du groupe R-CZ- de l'amine du cycle phénothiazine du composé de formule (II) à l'aide de moyens de déprotection n'impliquant pas l'utilisation de composés métalliques.

Par « moyens de déprotection n'impliquant pas l'utilisation de composés métalliques », on entend l'emploi de réactifs non métalliques, de solvants ne comportant pas de résidus métalliques (de préférence < 0,01 ppm), dans des réacteurs ne comportant pas de parties métalliques, comme des réacteurs émaillés par exemple.

Parmi les moyens utilisables pour la déprotection du groupe R-CZ- la demande décrit : les quinones, comme par exemple la 2,3-dichloro-5,6-dicyano-1,4-benzoquinone (DDQ), HNO₃, HClO₄, I₂, HCl, H₂SO₄, H₂O₂, un traitement par des rayonnements ultraviolets. Selon l'invention, on utilise une quinone pour cette étape, et de façon très préférentielle la 2,3-dichloro-5,6-dicyano-1,4-benzoquinone. Avantageusement on emploie les conditions de mise en œuvre du DDQ qui ont été décrites ci-dessus.

La méthode de déprotection du composé (II) en composé (I) permet d'aboutir à un composé (I) qui ne comporte pas d'impuretés métalliques additionnelles par rapport au produit (II). En outre, ces conditions de déprotection évitent la formation de produits de dégradation organique. En effet, les composés de formule (I) sont d'une stabilité limitée et l'utilisation de certaines conditions de traitement conduit à des dégradations, par exemple du bleu de méthylène en Azur A, B et C, qui sont ensuite difficilement séparables.

Le procédé de l'invention permet d'avoir accès à un composé de formule (I) dépourvu de contaminants métalliques et d'une haute pureté chimique, de façon fiable, reproductible et applicable à l'échelle industrielle. Ces qualités sont essentielles pour pouvoir fournir un produit de formule (I) de qualité pharmaceutique.

Notamment le procédé de préparation ou de purification de l'invention permet d'obtenir en quantités industrielles et de façon reproductible un bleu de méthylène ou chlorure de tétraméthylthionine qui comprend 0,02 µg/g ou moins de cadmium par g de bleu de méthylène.

Le procédé de l'invention donne accès à un bleu de méthylène ou chlorure de tétraméthylthionine ayant un taux de pureté supérieur à 97%, préférentiellement supérieur à 98%, encore mieux supérieur à 99%, mesurée en CLHP (chromatographie liquide haute performance) dans les conditions de la pharmacopée européenne 5.4 (édition d'avril 2006) et comprenant moins de 4,5 µg/g d'aluminium, avantageusement moins de 3 µg/g d'aluminium, encore plus avantageusement moins de 2,5 µg/g d'aluminium par g de bleu de méthylène.

Le procédé de l'invention donne accès à un bleu de méthylène ou chlorure de tétraméthylthionine ayant un taux de pureté supérieur à 97%, préférentiellement supérieur à 98%, encore mieux supérieur à 99%, mesurée en CLHP dans les conditions de la pharmacopée européenne 5.4 (édition d'avril 2006) et comprenant moins de 0,5 µg/g d'étain par g de bleu de méthylène.

Le procédé de l'invention donne accès à un bleu de méthylène ou chlorure de tétraméthylthionine ayant un taux de pureté supérieur à 97%, préférentiellement supérieur à 98%, encore mieux supérieur à 99%, mesurée en CLHP dans les conditions de la pharmacopée européenne 5.4 (édition d'avril 2006) et comprenant moins de 0,95µg/g de chrome, avantageusement moins de 0,90µg/g, encore mieux, moins de 0,80µg/g par g de bleu de méthylène.

Le procédé de l'invention est le seul à donner accès, en quantité industrielle, à un bleu de méthylène ou chlorure de tétraméthylthionine comprenant moins de 3% d'impuretés, préférentiellement moins de 2%, encore mieux moins de 1%, mesuré en CLHP dans les conditions de la pharmacopée européenne 5.4 (édition d'avril 2006) et un taux d'impuretés métalliques inférieur à 20 µg/g, avantageusement inférieur à 15 µg/g, encore plus avantageusement inférieur à 10 µg/g.

La demande décrit un composé de formule (I), à l'exclusion du bleu de méthylène ou chlorure de tétraméthylthionine et comportant un taux d'impuretés métalliques global inférieur à 100 µg/g, avantageusement inférieur à 50 µg/g, en particulier inférieur à 30 µg/g. La demande décrit que ce composé peut satisfaire une ou plusieurs des conditions suivantes :
- pureté supérieure à 97%, préférentiellement supérieure à 98%, encore mieux supérieure à 99%, mesurée en CLHP dans les conditions de la pharmacopée européenne 5.4 (édition d'avril 2006)
- taux d'aluminium inférieur à 5 µg/g, avantageusement inférieur à 4 µg/g, encore plus avantageusement inférieur à 3 µg/g,
- taux de cadmium inférieur à 0,1 µg/g, avantageusement inférieur à 0,05 µg/g, encore mieux inférieur à 0,02 µg/g,
- taux d'étain inférieur à 0,5 µg/g, avantageusement inférieur à 0,4 µg/g et encore plus avantageusement inférieur à 0,3 µg/g.

Le bleu de méthylène est utilisé depuis des décennies dans le traitement de diverses infections. Il est utilisé comme agent antiseptique, anti infectieux, comme antidote et comme agent de diagnostique. De façon récente son activité antivirale a été mise en évidence, et il pourrait être utilisé dans la préparation d'un médicament destiné à lutter contre une pathologie telle qu'une infection, notamment un choc septique, la présence de contaminants pathogéniques dans le sang ou le plasma, une réaction hémodynamique excessive, une infection par le HIV, le virus West Nile, le virus de l'hépatite C, la maladie d'Alzheimer, la malaria, le cancer du sein, les troubles maniaco-dépressifs.

Enfin il pourrait également être utilisé en cosmétique ou pour des produits destinés à une application ophtalmique.

Pour toutes ces applications thérapeutiques, et en particulier dans le contexte de la prévention et du traitement de la maladie d'Alzheimer, il est nécessaire de disposer d'un bleu de méthylène ayant un degré de pureté élevé et en particulier comportant très peu d'impuretés métalliques.

L'invention donne accès à un médicament comprenant un bleu de méthylène tel que décrit ci-dessus, dans un support pharmaceutiquement acceptable.

Le support et les quantités de bleu de méthylène à administrer sont bien connus de l'homme du métier.

L'invention a en outre pour objet un procédé de préparation d'un médicament comportant un Bleu de Méthylène, caractérisé en ce que ce procédé comporte au moins une étape de procédé telle que décrite ci-dessus, en particulier une étape de purification du composé de formule (I) et/ou une étape de déprotection du composé (II) en (I).

### PARTIE EXPERIMENTALE

### 1- Synthèse du Benzoyl leuco bleu de Méthylène (Etape A)

Dans un réacteur double enveloppe de 120 L muni d'une agitation et sous azote on introduit :
- 80 L d'eau distillée,
- 4,2 kg (10,7 moles) de bleu de méthylène commercialisé par la société LEANCARE LTD sous la référence CI 52015, comprenant d'importantes quantités d'impuretés métalliques (Al, Fe, Cu, Cr).

On laisse 15 min sous agitation puis on ajoute 6,9 kg de sodium hydrosulfite Na₂S₂O₄ en solution aqueuse à 85%. La coloration vire du bleu vers le beige. On laisse 45 min supplémentaires sous agitation, puis on ajoute 2,69 kg de soude sous forme de pastilles. La température de la réaction est maintenue entre 18 et 20°C. La durée d'addition est de 30 min et on laisse sous agitation 30 min supplémentaires. Ensuite on ajoute goutte-à-goutte 7,90 L de chlorure de benzoyle. Le mélange réactionnel vire vers une coloration vert-beige. La durée d'addition est de 2h et ensuite on laisse sous agitation pendant 20 h.

### Traitement :

Après arrêt de l'agitation on laisse décanter 15 min et on aspire le surnageant. On ajoute 80 L d'eau (25 volumes) et après agitation et décantation on aspire de nouveau le surnageant. On ajoute 24 L d'EtOH et après une agitation d'environ 5 min on ajoute 16 L d'eau. Après avoir agité pendant 15 min, le mélange est filtré sur recette. Cette opération est répétée 3 fois. Après séchage on obtient 2,9 kg (Rdt : 66%) de benzoyl leuco bleu de méthylène.

### 2- Purification

On utilise 4,25 kg de benzoyl leuco bleu de méthylène issu de la première étape, solubilisé dans 30 L de CH₂Cl₂. On filtre sur 3 parts de silice (Merck Gerudan Si60) (11,5 kg) et 0,5kg de sable de Fontainebleau on rince avec 30 litres de CH₂Cl₂. On élimine CH₂Cl₂ par évaporation sous vide. On ajoute 6 L d'éthanol. On laisse sous agitation à froid puis on filtre sur recette. On sèche sous vide. On obtient 3,4 kg de benzoyl leuco bleu de méthylène purifié (Rdt : 80%).

### Pureté: +99 % CLHP

Métaux : le contenu en métaux (en µg/g) est donné pour 3 essais dans le tableau 1.

**Tableau 1**

| Essai | Essai 1 | Essai 2 | Essai 3 |
|---|---|---|---|
| Al | **0,5** | **0,5** | **0,1** |
| Cu | **0** | **0** | **0,4** |
| Fe | **0** | **0** | **0,1** |
| Zn | **0,9** | **0,7** | **0,5** |
| Ni | **0,1** | **0,1** | **0,1** |
| Cr | **0,3** | **0,3** | **0,03** |
| Mo | **0,1** | **0,1** | **0,1** |
| Mn | **0,02** | **0** | **0** |
| Sn | **0,5** | **0,4** | **0,5** |
| Pb | **5** | **3,2** | **2,4** |
| Cd | **0,2** | **0,2** | **0,07** |

### 3- Débenzoylation

Dans un réacteur double enveloppe émaillé de 100 l à température ambiante, on introduit :
- 45 L d'acétonitrile (ACN)
- 1,6 kg du benzoyl leuco bleu de méthylène issu de la seconde étape et on place sous agitation. On laisse agiter 30 min à température ambiante puis on baisse la température à -18°C. On ajoute en une portion 950 g de DDQ solubilisés dans 4 L d'ACN. On laisse agiter 2h à -18°C. On filtre. On obtient un complexe du 3,7-bis(diméthyl amino)phénothiazine avec le DDQ qui est utilisé directement dans l'étape suivante.

### 4- Salification

Dans le réacteur double enveloppe émaillé, on réintroduit le gâteau issu de la troisième étape en plusieurs morceaux. On ajoute 4 L d'AcOEt. On laisse tourner pendant 30 min à température ambiante. On baisse la température à -18°C. On ajoute 2,5 kg d'HCl dans 16 L d'AcOEt (solution 4N) . On agite 2h à -18°C. On filtre puis on réintroduit le gâteau dans le réacteur. On ajoute 30 L d'AcOEt à -18°C et on filtre à nouveau.

### 5- Neutralisation

On ajoute 30 L d'acétone puis une solution de 200 g de NaOH solubilisés dans 500 ml d'eau. On filtre. On introduit le produit issu de la quatrième étape dans le réacteur avec 30 L d'acétone. On agite le milieu pendant 1h à température ambiante. Le pH est de 4,0. On filtre. On laisse sous vide sur la recette.

### 6- Purification et hydratation

Dans un réacteur émaillé de 40 L sous N₂, à température ambiante, on a introduit 1,9 kg du produit de la cinquième étape et 30 L du mélange 50/50 CH₂Cl₂/EtOH. On chauffe au reflux (43°C). On filtre à chaud avec un filtre microfibre (Whatman GF/D). Cette opération est effectuée 2 fois. On nettoie le réacteur à l'eau déminéralisée. On réintroduit le filtrat dans le réacteur. On distille sous vide à 28°C 24 litres de solvant (3h). Le milieu est remis dans le réacteur. On ajoute 1 L d'eau microfiltrée. On refroidit à -18°C. On ajoute 40 L d'AcOEt et on laisse la nuit sous agitation à froid. On filtre. On empatte avec 10 L d'AcOEt. On obtient 1,4 kg de bleu de méthylène purifié sous la forme trihydratée.

Les impuretés métalliques sont analysées et rapportées dans le tableau 2.

**Tableau 2**

| élément | Quantité (µg/g) |
|---|---|
| Al | **1,3** |
| Cu | **0,5** |
| Fe | **1,9** |
| Zn | **1,7** |
| Ni | **0,5** |
| Cr | **0,8** |
| Mo | **0,2** |
| Mn | **0,08** |
| Sn | **0,4** |
| Pb | **0,1** |
| Cd | **0,04** |

## Revendications

1. Procédé de préparation d'un composé répondant à la formule (I) ci-dessous : 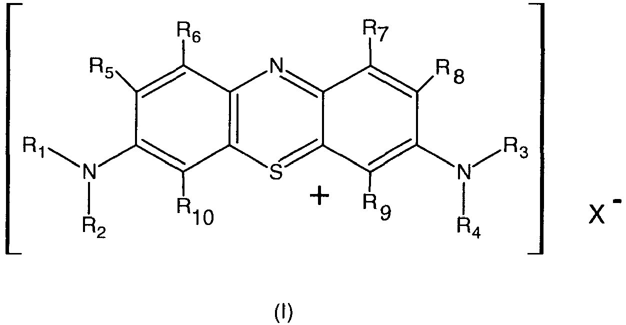 dans laquelle chacun de R₁, R₂, R₃, R₄, R₅, R₆, R₇, R₈, R₉, R₁₀ peut être choisi, indépendamment des autres, parmi le groupe constitué de :
- l'atome d'hydrogène,
- les groupements alkyle en C₁-C₆, linéaires ramifiés ou cycliques, saturés ou insaturés, éventuellement substitués par une ou plusieurs fonctions choisies parmi un atome d'halogène, une fonction alcoxy en C₁-C₆, alkyloxycarbonyle en C₁-C₆, - CONH₂,
- les groupements aryle éventuellement substitués par une ou plusieurs fonctions choisies parmi : un alkyle en C₁-C₄, un atome d'halogène, une fonction alcoxy en C₁-C₆, alkyloxycarbonyle en C₁-C₆, -CONH₂,
en outre, chacun de R₅, R₆, R₇, R₈, R₉, R₁₀ peut être choisi, indépendamment des autres, parmi les atomes d'halogène : F, Cl, Br, I,
X⁻ représente un anion organique ou inorganique,
**caractérisé en ce qu'**il comporte au moins une étape au cours de laquelle un composé de formule (II) : 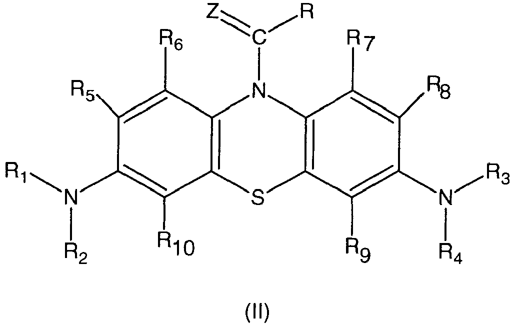 dans laquelle R représente un groupement choisi parmi :
- un groupement phényle ou benzyle, éventuellement substitués par une ou plusieurs fonctions choisies parmi : un alkyle en C₁-C₄, un atome d'halogène, un halogénoalkyle en C₁-C₄, un groupement nitro,
- un groupement alkyle en C₁-C₈, linéaire, ramifié ou cyclique,
- un groupement alkyl amino en C₁-C₈,
- un groupement alcoxy en C₁-C₈,
- un groupement phényloxy ou benzyloxy éventuellement substitués sur le noyau aromatique par une ou plusieurs fonctions choisies parmi : un alkyle en C₁-C₄, un atome d'halogène, un halogénoalkyle en C₁-C₄, un groupement nitro,
Z représente un atome choisi parmi O et S,
est soumis à une étape de déprotection de l'amine du cycle phénothiazine à l'aide de moyens de déprotection n'impliquant pas l'utilisation de composés métalliques, le réactif de déprotection étant choisi parmi : les quinones.

2. Procédé selon la revendication 1, dans lequel le réactif de déprotection est la 2,3-dichloro-5,6-dicyano-1,4-benzoquinone (DDQ).

3. Procédé selon la revendication 2, dans lequel on emploie de 0,80 à 1,1 équivalents molaires de DDQ par rapport au composé (II).

4. Procédé selon l'une quelconque des revendications 1 à 3 qui est mis en œuvre dans un solvant ou un mélange de solvants comportant moins de 0,01 ppm de résidus métalliques, et dans des réacteurs ne comportant pas de parties métalliques, comme des réacteurs émaillés.

5. Procédé selon l'une quelconque des revendications 1 à 4 dans lequel la réaction de déprotection se fait à une température comprise entre -40°C et -5°C.

6. Procédé selon l'une quelconque des revendications 1 à 5 dans lequel la réaction de déprotection est faite dans un solvant choisi parmi : l'acétate d'éthyle, l'acétonitrile, le tétrahydrofurane, l'acétone

7. Procédé selon l'une quelconque des revendications précédentes, dans lequel le composé de formule (I) est le chlorure de tétraméthylthionine ou bleu de méthylène.

8. Procédé selon l'une quelconque des revendications 1 à 7 qui comporte au moins une étape au cours de laquelle le composé de formule (II) : 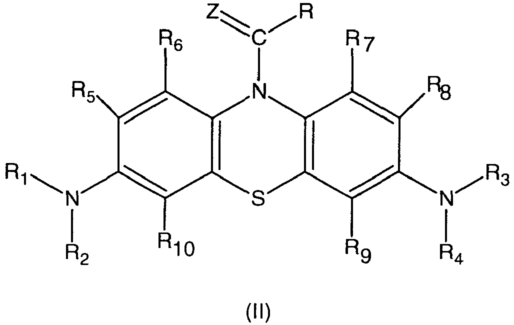 est soumis à une étape de purification dans des conditions permettant de séparer des composés métalliques des composés de formule (II).

9. Procédé selon la revendication 8, dans lequel l'étape de purification comporte au moins une filtration sur un support susceptible de retenir les composés métalliques.

10. Procédé selon la revendication 9, dans lequel le support de filtration est choisi parmi : un gel de silice, un gel d'alumine neutre, basique ou acide, une membrane microporeuse, une résine greffée par des groupements capteurs de métaux, des fibres greffées par des groupements capteurs de métaux.

11. Procédé selon l'une quelconque des revendications 8 à 10, dans lequel l'étape de purification comporte au moins une cristallisation.

12. Procédé selon l'une quelconque des revendications précédentes, qui comporte en outre une étape d'échange d'ion, par traitement avec HCl.

13. Procédé selon l'une quelconque des revendications 8 à 12, dans lequel on obtient un Bleu de méthylène (chlorure de 3,7-bis(diméthylamino)phénothiazine-5-ium) comprenant moins de 3% d'impuretés et un taux de métaux inférieur à 20 µg/g, avantageusement inférieur à 15 µg/g, encore plus avantageusement inférieur à 10 µg/g.

14. Procédé de préparation d'un médicament comportant du Bleu de Méthylène, ce procédé comprenant la préparation du Bleu de Méthylène est par un procédé suivant l'une quelconque des revendications 1 à 13.

## Patentansprüche

1. Verfahren zur Herstellung einer Verbindung der nachstehenden Formel (I): 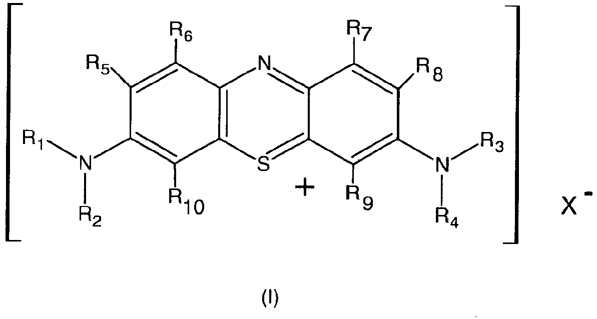 worin R₁, R₂, R₃, R₄, R₅, R₆, R₇, R₈, R₉ und R₁₀ jeweils unabhängig voneinander aus der Gruppe bestehend aus:
- einem Wasserstoffatom,
- gesättigten oder ungesättigten, linearen, verzweigten oder cyclischen C₁-C₆-Alkylgruppen, die gegebenenfalls durch eine oder mehrere Funktionen, die aus einem Halogenatom und einer C₁-C₆-Alkoxy-, C₁-C₆-Alkyloxycarbonyl- oder -CONH₂-Funktion ausgewählt sind, substituiert sind,
- Arylgruppen, die gegebenenfalls durch eine oder mehrere Funktionen, die aus einem C₁-C₄-Alkyl, einem Halogenatom und einer C₁-C₆-Alkoxy-, C₁-C₆-Alkyloxycarbonyl- oder -CONH₂-Funktion ausgewählt sind, substituiert sind, ausgewählt sein können und außerdem R₅, R₆, R₇, R₈, R₉ und R₁₀ jeweils unabhängig voneinander aus den Halogenatomen, F, Cl, Br und I, ausgewählt sein können,
X⁻ für ein organisches oder anorganisches Anion steht,
**dadurch gekennzeichnet, dass** es mindestens einen Schritt umfasst, bei dem man eine Verbindung der Formel (II): 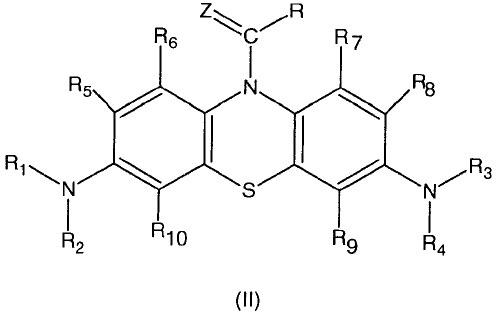 worin R für eine Gruppe steht, die aus:
- einer Phenyl- oder Benzylgruppe, die gegebenenfalls durch eine oder mehrere Funktionen, die aus einem C₁-C₄-Alkyl, einem Halogenatom, einem C₁-C₄-Halogenalkyl und einer Nitrogruppe ausgewählt sind, substituiert ist,
- einer linearen, verzweigten oder cyclischen C₁-C₈-Alkylgruppe,
- einer C₁-C₈-Alkylaminogruppe,
- einer C₁-C₈-Alkoxygruppe,
- einer Phenyloxy- oder Benzyloxygruppe, die gegebenenfalls am aromatischen Kern durch eine oder mehrere Funktionen, die aus einem C₁-C₄-Alkyl, einem Halogenatom, einem C₁-C₄-Halogenalkyl und einer Nitrogruppe ausgewählt sind, substituiert ist,
Z für ein aus O und S ausgewähltes Atom steht,
ausgewählt ist,
einem Schritt der Entschützung des Amins des Phenothiazinrings mit Hilfe von Entschützungsmitteln, die keine Verwendung von Metallverbindungen mit sich bringen, unterwirft, wobei das Entschützungsmittel aus Chinonen ausgewählt wird.

2. Verfahren nach Anspruch 1, wobei es sich bei dem Entschützungsreagenz um 2,3-Dichlor-5,6-dicyano-1,4-benzochinon (DDQ) handelt.

3. Verfahren nach Anspruch 2, wobei man 0,80 bis 1,1 Moläquivalente DDQ, bezogen auf die Verbindung (II), einsetzt.

4. Verfahren nach einem der Ansprüche 1 bis 3, das in einem Lösungsmittel oder einem Lösungsmittelgemisch mit weniger als 0,01 ppm Metallrückständen und in Reaktoren ohne Metallteile, wie in emaillierten Reaktoren, durchgeführt wird.

5. Verfahren nach einem der Ansprüche 1 bis 4, wobei die Entschützungsreaktion bei einer Temperatur zwischen -40 °C und -5 °C erfolgt.

6. Verfahren nach einem der Ansprüche1 bis 5, wobei die Entschützungsreaktion in einem aus Essigsäureethylester, Acetonitril, Tetrahydrofuran und Aceton ausgewählten Lösungsmittel erfolgt.

7. Verfahren nach einem der vorhergehenden Ansprüche, wobei es sich bei der Verbindung der Formel (I) um Tetramethylthioninchlorid oder Methylenblau handelt.

8. Verfahren nach einem der Ansprüche 1 bis 7, das mindestens einen Schritt umfasst, bei dem man eine Verbindung der Formel (II): 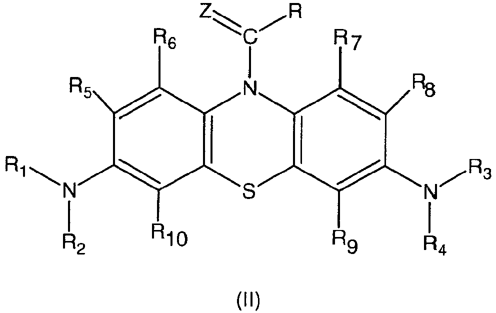 einem Reinigungsschritt unter Bedingungen unterwirft, die eine Abtrennung von Metallverbindungen von den Verbindungen der Formel (II) erlauben.

9. Verfahren nach Anspruch 8, wobei der Reinigungsschritt mindestens eine Filtration über einen Träger, der Metallverbindungen zurückhalten kann, umfasst.

10. Verfahren nach Anspruch 9, wobei der Filtrationsträger aus einem Kieselgel, einem neutralen, basischen oder sauren Aluminiumoxidgel, einer mikroporösen Membran, einem mit Metalleinfanggruppen gepfropften Harz und mit Metalleinfanggruppen gepfropften Fasern ausgewählt ist.

11. Verfahren nach einem der Ansprüche 8 bis 10, wobei der Reinigungsschritt mindestens eine Kristallisation umfasst.

12. Verfahren nach einem der vorhergehenden Ansprüche, das außerdem einen Ionenaustauschschritt durch Behandlung mit HCl umfasst.

13. Verfahren nach einem der Ansprüche 8 bis 12, wobei man ein Methylenblau (3,7-Bis(dimethylamino)phenothiazin-5-iumchlorid) mit weniger als 3% Verunreinigungen und einem Gehalt an Metallen von weniger als 20 µg/g, vorteilhafterweise weniger als 15 µg/g und noch vorteilhafter weniger als 10 µg/g erhält.

14. Verfahren zur Herstellung eines Medikaments, das Methylenblau umfasst, wobei dieses Verfahren die Herstellung des Methylenblaus durch ein Verfahren gemäß einem der Ansprüche 1 bis 13 umfasst.

## Claims

1. A process for preparing a compound corresponding to formula (I) below: 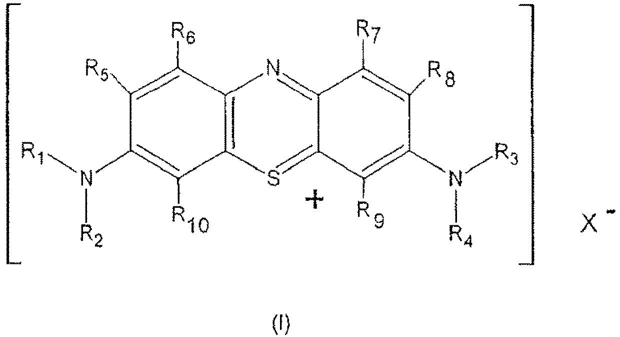 in which each of R₁, R₂, R₃, R₄, R₅, R₆, R₇, R₈, R₉ and R₁₀ may be chosen, independently of the others, from the group constituted of:
- a hydrogen atom,
- saturated or unsaturated, linear, branched or cyclic C₁-C₆ alkyl groups, optionally substituted with one or more functions chosen from a halogen atom, and a C₁-C₆ alkoxy, C₁-C₆ alkoxycarbonyl or -CONH₂ function,
- aryl groups optionally substituted with one or more functions chosen from: a C₁-C₄ alkyl, a halogen atom, and a C₁-C₆ alkoxy, C₁-C₆ alkyloxycarbonyl or -CONH₂ function,
in addition, each of R₅, R₆, R₇, R₈, R₉ and R₁₀ may be chosen, independently of the others, from the halogen atoms: F, Cl, Br and I,
X⁻ represents an organic or inorganic anion,
**characterized in that** it comprises at least one step during which a compound of formula (II): 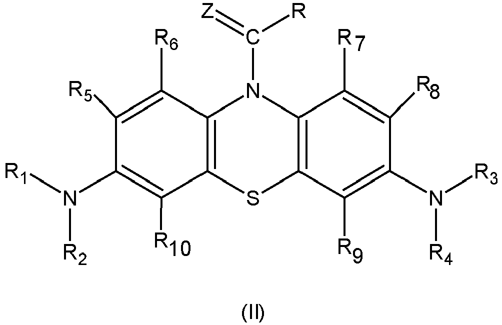 in which R represents a group chosen from:
- a phenyl or benzyl group, optionally substituted with one or more functions chosen from: a C₁-C₄ alkyl, a halogen atom, a C₁-C₄ haloalkyl and a nitro group,
- a linear, branched or cyclic C₁-C₈ alkyl group,
- a C₁-C₈ alkylamino group,
- a C₁-C₈ alkoxy group,
- a phenyloxy or benzyloxy group optionally substituted on the aromatic nucleus with one or more functions chosen from: a C₁-C₄ alkyl, a halogen atom, a C₁-C₄ haloalkyl and a nitro group,
Z represents an atom chosen from O and S,
is subjected to a step for deprotection of the amine of the phenothiazine ring by a means that does not imply the use of metal reactants, the deprotection reactant being chosen from: quinones.

2. The process as claimed in claim 1, in which the deprotection means is 2,3-dichloro-5,6-dicyano-1,4-benzoquinone (DDQ).

3. The process as claimed in claim 2, which is implemented with from 0.80 to 1.1 molar equivalents of DDQ relative to the compound (II).

4. The process as claimed in anyone of claim 1 to 3, which is implemented in a solvent or a mixture of solvents comprising less than 0.01 ppm metal residues, in reactors not comprising any metal parts, for instance enameled reactors.

5. The process as claimed in anyone of claim 1 to 4, wherein the step for deprotection is implemented at a temperature of between -40°C and -5°C.

6. The process as claimed in anyone of claim 1 to 5, wherein the step for deprotection is implemented in a solvent chosen from: ethyl acetate, acetonitrile, tetrahydrofuran and acetone.

7. The process as claimed in any one of the preceding claims, wherein the compound of formula (I) is methylthioninium chloride or methylene blue.

8. The process as claimed in any one of claims 1 to 7, which comprises at least one step during which a compound of formula (II): 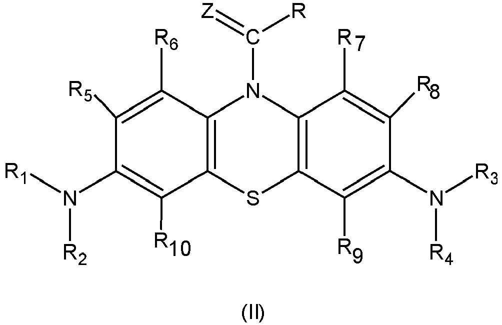 is subjected to a purification step under conditions which make it possible to separate metal compounds from the compounds of formula (II).

9. The process as claimed in claim 8, wherein the purification step comprises at least one filtration through a support capable of retaining the metal compounds.

10. The process as claimed in claim 9, wherein the filtration support is chosen from: a silica gel, a neutral, basic or acidic alumina gel, a microporous membrane, a resin grafted with metal-capturing groups, and fibers grafted with metal-capturing groups.

11. The process as claimed in any one of claims 8 to 10, wherein the purification step includes at least one crystallization.

12. The process as claimed in any one of the preceding claims, which further comprises a step for ion exchange, by treatment with HCl.

13. The process as claimed in any one of claims 8 to 12, wherein is obtained a Methylene blue (3,7-bis(dimethylamino)phenothiazin-5-ylium chloride) comprising less than 3% impurities and a level of metals inferior to 20 microgram per gram, advantageously inferior to 15 microgram per gram, even more advantageously inferior to 10 microgram per gram.

14. A process for preparing a medicament comprising methylene blue, this process comprising the preparation of methylene blue by a process according to any one of claims 1 to 13.
